# EUROPÄISCHE PATENTSCHRIFT

(11) **EP 1 159 936 B1**
(45) Veröffentlichungstag und Bekanntmachung des Hinweises auf die Patenterteilung: **07.05.2008**
(21) Anmeldenummer: 01107579.3
(22) Anmeldetag: 27.03.2001
(51) Int. Cl.: A61F 2/30

(54) **Dämpfer für Rotationsbewegungen bei Prothesen**
Damper for rotating movements of prothesis
Amortisseur pour des mouvements de rotation des prothèses

(30) Priorität: 27.05.2000 DE 10025921
(43) Veröffentlichungstag der Anmeldung: 05.12.2001
(73) Patentinhaber: OHIO WILLOW WOOD COMPANY, Mount Sterling, OH 43143 (US)
(72) Erfinder: Lührs, Bernd, 33142 Büren (DE); Theile, Frank, 34519 Diemelsee Adorf (DE); Griesser, Michael, 33154 Salzkotten (DE)
(74) Vertreter: Holland, Ralf

(56) Entgegenhaltungen:
- GB-A- 108 003
- US-A- 3 706 465
- US-A- 5 728 175

## Beschreibung

Die Erfindung betrifft einen Dämpfer für Rotationsbewegungen bei Prothesen, insbesondere bei Ober- und Unterschenkelprothesen.

In der Prothetik ist ein Rotationsbewegungen und Stöße dämpfendes Federsystem bekannt, bei dem zwei gegeneinander axial federnd gelagerte Platten bei einer Drehbewegung um eine Hochachse, aufgrund der Geometrie der sich gegenüberliegenden Plattenoberflächen, sich gegen die Kraft von Federn axial mit Bezug auf die Hochachse voneinander entfernen.

Eine solche Technik ist ferner aus der US 3,706,465 bekannt, in der ein Dämpfer erläutert wird, bei dem zwei im wesentlichen gleichartig ausgebildete Platten sich über drei elastomere Stempel axial abstützen. Da die Stempel in Umfangsrichtung in Ausnehmungen der oberen wie auch in Ausnehmungen in der unteren Platte festgelegt sind, ist der Drehwinkel der Platte gegeneinander grundsätzlich eingeschränkt. Neben der Federwirkung durch die elastomeren Stempel ist weiter eine Spiralfeder vorgesehen, die um eine zentrale Achse gelegt einerends gegen eine Anschlagfläche der oberen Platte und andernends gegen eine Anschlagfläche der unteren Platte sich abstützt.

Eine solche Dämpfung von Drehbewegungen, einhergehend mit axialen Verschiebungen, führt regelmäßig zu erheblichen Scherkräften zwischen einem Amputationsstumpf und der Prothese, ggfls. innerhalb der Prothese selbst, wenn der Dämpfer dort angeordnet ist. Diese auftretenden Kräfte bewirken einen unnatürlichen Bewegungsablauf, den es grundsätzlich in der Prothetik zu vermeiden gilt. Daneben ist eine solche Dämpfung aufgrund reibender oder rollender Anlage von Oberflächen stets verschleißanfällig.

Bei dem in der GB 108003 erläuterten Dämpfer wirkt eine Spiraldruckfeder zwischen einer Anschlagfläche einer unteren Platte und einer Anschlagfläche einer oberen Platte. Hierzu ist zwischen den Anschlagflächen und den Platten ein Ringraum ausgebildet, in dem die Feder frei eingelegt ist und sich endseitig an den Anschlagflächen abstützt.

Bei der aus der US 5,728,175 bekannten Prothese ist ein Dämpfer vorgesehen, bei dem zwei Torsionsfedern auf einem Zapfen jeweils eines Gehäusedeckels geführt sind, welche Torsionsfedern einerends gehäusedeckelfest und andernends an der körperlich ausgebildeten Drehachse festgelegt sind. Aufgrund dieser Konstruktionsweise wird bei einem Tordieren der Federn sich deren Durchmesser ändern, womit Reaktionskräfte zwischen den zwei Gehäusedeckeln und der Achse auftreten.

Vor diesem technischen Hintergrund macht die Erfindung es sich zur Aufgabe, einen Dämpfer für Rotationsbewegungen bei Prothesen zur Verfügung zu stellen, der einen weitestgehend natürlichen Bewegungsablauf ermöglicht und durch den insbesondere axiale Bewegungen und durch eine solche axiale Bewegung hervorgerufene Kräfte vermieden sind.

Diese technische Problematik wird durch den Gegenstand nach der Erfindung gelöst, wobei bei dem Dämpfer nach Anspruch 1 auf wenigstens einen gehäuseoberteilteilfesten, zwei radiale Anschlagflächen ausbildenden Anschlag abgestellt ist, an die wenigstens ein an der Aufnahme festgelegtes Federelement angeschlossen ist, dessen Kraft die Drehbewegung dämpft und das Gehäuseoberteil nach einem Verdrehen gegenüber der Aufnahme in eine Neutrallage zurückführt.

Bei dem Dämpfer nach der Erfindung wird die Kraft des Federelementes nur in einer Ebene, im wesentlichen radial an dem Anschlag angreifen und die radiale Bewegung dämpfen. Eine axiale Bewegung in Bezug auf die Hochachse ist konstruktionsbedingt nicht möglich. Zweckmäßigerweise werden, da naturgegeben Drehbewegungen in zwei Richtungen um eine Hochachse möglich sind, die am Anschlag angreifenden Kräfte drehsymmetrisch und damit in jede Drehbewegung gleich groß vorgesehen sein. Infolge werden die um die Hochachse möglichen Drehbewegungen in beide Richtungen gleichermaßen gedämpft.

In konstruktiver Ausgestaltung hat es sich bewährt, daß der Anschlag in einer zumindest abschnittsweise ringartigen Nut in der Aufnahme geführt ist. In Umfangsrichtung links und rechts können dann bspw. zwei Druckfedern angeordnet sein, die als Federelemente ausgebildet den Anschlag radial zwischen sich spielfrei halten und die andernends aufnahmefest angebunden sind, indem sie sich gegen die Stirnwände dieser Nut abstützen.

In konstruktiver bevorzugter Ausgestaltung ist jedoch vorgesehen, daß die ringartige Nut durchgehend ringförmig, insbesondere kreisförmig ausgebildet ist, daß dann in die ringartige Nut eine Ringfeder eingelegt ist, deren Enden unter Einfassen des Anschlages sich in einer Neutrallage überkreuzend an Wänden von radial der Nut vorstehenden Taschen abstützen. Es ist damit der Anschlag radial von den sich überkreuzenden Federenden eingefaßt. Bei einem Verdrehen wird sich ein Ende an einer Wand einer ersten Tasche abstützen, währende das andere Federende mit anliegendem Anschlag sich innerhalb der Tasche bewegen kann.

Die radial den Wänden, an denen in der Neutralstellung sich die Federenden abstützen, gegenüberliegenden Wände der Taschen werden in jedem Fall dann die Auslenkung des Dämpfers begrenzen. Dabei ist an einen Umfangswinkel von etwa 50° bei den Taschen gedacht und an einen maximale Drehwinkel von etwa 130°, wobei durch einen zwischen den Taschen verbleibender Steg von einem Umfangswinkel von 30° für eine Vorspannung der Ringfeder gesorgt ist.

In Ausgestaltung der Erfindung kann weiter vorgesehen sein, daß das Einfassen des Anschlages durch elastische Puffer gedämpft erfolgt, wie auch das Abstützen der Enden der Ringfeder innerhalb der Taschen. Durch diese Maßnahme wird vermieden, daß ein harter metallischer Anschlag erfolgt, der z. B. auch zu hören wäre. Solche Puffer können einfache elastische Auflagen auf den entsprechenden Wänden sein oder können z. B. Gummipuffer gesondert ausgebildet und in die Wände der Taschen bzw. des Anschlages eingesetzt sein. Auch kann an eine Ummantelung der Federenden selbst gedacht werden, die anschlagsmildernd wirkt.

Ist daran gedacht, daß ein Anschlag radial zwischen zwei Federelementen spielfrei gehalten ist, die andernends aufnahmefest angebunden sind, ist bevorzugt, daß diese zwei Federelemente als Druckfederelemente ausgebildet sind, beispielsweise als Druckfedern oder als Gummistücke. Es hat diese Maßnahme den Vorteil, daß eine Befestigung der Federelemente an dem Anschlag und an der Aufnahme nicht nötig ist. Zwar kann auch ein Paar von Zugfedern bspw. Verwendung finden, jedoch sind diese dann endseitig zum einen an dem Anschlag und zum anderen an der Aufnahme zu befestigen.

Vorteilhaft einsetzbar sind aus einem Elastomer bestehenden Druckkörpern, die die zwei einen Anschlag einfassenden Federelemente ausbilden. Insbesondere durch eine geeignete Auswahl des Elastomers ist nahezu eine beliebige Dämpfungscharakteristik einstellbar. Bevorzugt kann hierdurch eine sehr progressive Dämpfung durch die zwei Federelemente weiter erreicht werden.

In bevorzugter Ausgestaltung ist vorgesehen, daß sich die zwei Federelemente in an die Nut angeschlossenen Taschen abstützen, die insbesondere tangential an den den Anschlag führenden Nutabschnitt angesetzt sind. In Folge dieser Maßnahme ist es möglich, die Nut auch ringförmig umlaufend auszubilden und dennoch die Federelemente an der, der an dem Anschlag anliegenden Stirnseite gegenüberliegenden Stirnseite, abzustützen oder zu befestigen.

In weitere konstruktiver Ausgestaltung ist vorgesehen, daß die die zwei Federelemente abstützenden Taschen eine einstellbare Tiefe aufweisen. Zum einen kann damit die spielfrei Halterung des Anschlages zwischen den beiden Federelementen leicht eingestellt werden. Darüber hinaus ist es möglich, auf die Federelemente eine Vorspannung aufzubringen und so die Dämpfungskraft des Dämpfers nach der Erfindung individuell für eine Prothesenträger anzupassen.

Hierbei kann daran gedacht sein, daß die Aufnahme jeweils eine in der Tasche in tangentialer Verlängerung mündende Durchbrechung für das Einbringen einer Schraube aufweist, deren Eindrehtiefe in die Durchbrechung die Tiefe der Tasche bestimmt. Es kann die Durchbrechung eine einfache Bohrung mit Gewinde sein, in die lediglich eine Schraube eingedreht ist. Es wird sich dann das entsprechende Federelement auf dem gewindeseitigen Ende der Schraube abstützen. Es kann jedoch auch daran gedacht sein, daß zwischen dem Federelement und dem abstützende Ende der Schraube eine Platte vorgesehen ist, so daß sichergestellt ist, daß das Ende der Schraube nicht in das Federelement eindringt, wenn dieses bspw. ein stabförmiger Druckkörper aus einem Elastomer ist.

In einer bevorzugten Ausführungsform des Dämpfers nach der Erfindung sind am Gehäuseoberteil zwei sich diametral gegenüberliegende Anschläge vorgesehen, von denen einer radial zwischen zwei Federelementen spielefrei gehalten ist und der andere zwischen den sich überkreuzenden Enden einer Ringfeder. Durch diese Maßnahme werden die vorstehend erläuterten Arten der Dämpfung einer Rotationsbewegung miteinander kombiniert. Eine äußerst natürlich wirkende Dämpfung einer Rotationsbewegung in der Protehtik ist damit gegeben. Auch ist die Rückstellung nach einer Auslenkung in eine Neutrallage durch diesen Dämpfer nach der Erfindung sicher und sehr natürlich wirkend ermöglicht. Dies insbesondere durch die Kombination zweier sehr progressiv wirkender Federelemente aus einem Elastomer sowie der linear wirkenden Ringfeder.

Um Reibungskräfte zwischen der Aufnahme und dem Gehäuseoberteil weitgehend auszuschließen, ist vorgesehen, daß das Gehäuseoberteil mittels eines Axiallagers an der Aufnahme drehbar gelagert ist. Durch das Vermeiden von Reibungskräften wird Verschleiß bei dem Dämpfer nach der Erfindung vermieden. Hierbei ist insbesondere daran gedacht, daß das Axiallager als Nadellager ausgebildet ist, das eine äußerst geringe Bauhöhe mit Bezug auf die Hochachse aufweist.

Die Erfindung wird anhand der Zeichnung näher erläutert, in der lediglich ein Ausführungsbeispiel dargestellt ist. In der Zeichnung zeigt:
- Fig. 1: eine isometrische Darstellung eines Dämpfers nach der Erfindung,
- Fig. 2: eine Unteransicht,
- Fig. 3: eine erste Seitenansicht,
- Fig. 4: eine zweite Seitenansicht,
- Fig. 5: eine Draufsicht und
- Fig. 6: eine teilweise gebrochene Darstellung des Dämpfers nach Fig. 1,
- Fig. 7: eine Draufsicht auf eine Aufnahme des Dämpfers nach Fig. 1,
- Fig. 8: eine isometrische Darstellung der Aufnahme,
- Fig. 9: eine Seitenansicht der Aufnahme,
- Fig. 10: einen diametralen Schnitt durch die Aufnahme gemäß der Linie X, X in Fig. 9,
- Fig. 11: einen Schnitt gemäß der Linie XI, XI in Fig. 7,
- Fig. 12: eine Draufsicht auf eine Aufnahme mit schematisch dargestellten eingelegten Federelementen und gebrochen gezeichneten Anschlägen,
- Fig. 13: einen oberen Gehäusedeckel des Gehäuseoberteils in einer Unteransicht,
- Fig. 14: einen Schnitt durch den Gehäusedeckel gem. Fig. 13,
- Fig. 15: einen Anschläge aufweisenden Ring des Gehäuseoberteils in einem Schnitt gem. der Linie XV, XV in Fig. 16,
- Fig. 16: eine Unteransicht des die Anschläge aufweisenden Ringes,
- Fig. 17: eine unterseitige, isometrische Darstellung des Ringes und
- Fig. 18: einen oberen Deckel des Dämpfers in einem Schnitt.

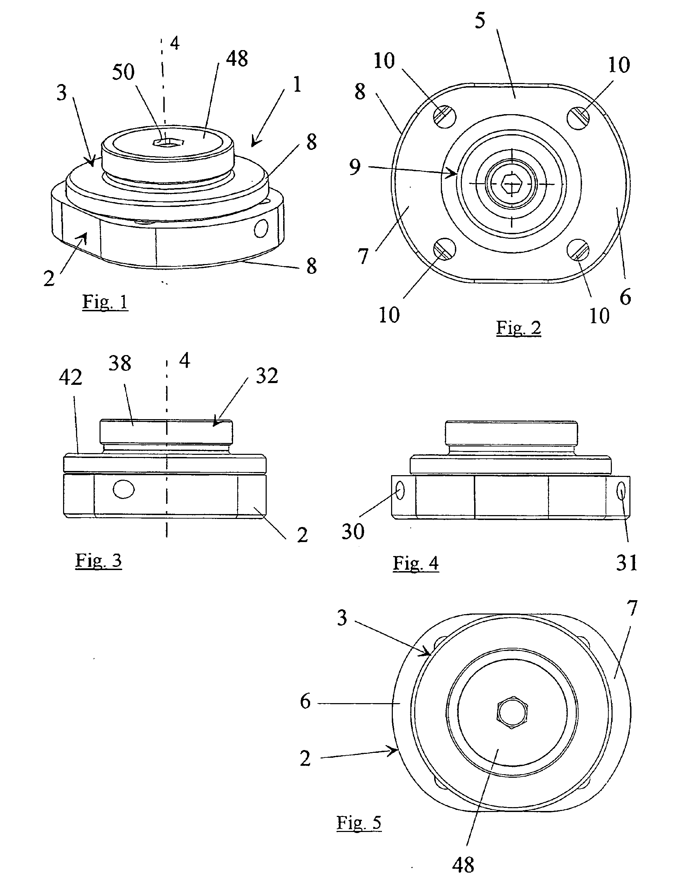

Der in den Figuren 1 bis 5 dargestellte Dämpfer 1 nach der Erfindung weist eine untere Aufnahme 2 auf, an der ein Gehäuseoberteil 3 um eine Hochachse 4 drehbar angeschlossen ist. Die Aufnahme 2 ist von einem zwei gegenüberliegende halbkreisförmige, ein Rechteck 5 einfassende Abschnitte 6, 7 aufweisenden Querschnitt. Das Gehäuseoberteil 3 besitzt einen kreisrunden Querschnitt mit einem Durchmesser, der dem der halbkreisförmigen Abschnitte 6, 7 entspricht. Diese halbkreisförmigen Abschnitte 6, 7 stehen damit um das halbe Maß der kleineren Breitenabmessung des Rechtecks 5 vor.

Alle äußeren Kanten der Aufnahme 2 und des Gehäuseoberteils 3 weisen Abfasungen 8 oder Abrundungen auf, die eine Verletzungs- oder Beschädigungsgefahr an scharfen Kanten mindern.

Unterseitig, vergleiche Fig. 2, ist die Aufnahme 2 mit einer ihrem Verwendungszweck angepaßten Profilierung 9 für ein Ansetzen bspw. an eine Prothese versehen. Hier sind weiter vier rotationssymmetrisch zu der Hochachse 4 angeordnete Schraubenlöcher 10 gezeigt, die einen festen und verdrehungsfreien Anschluß an eine solche Prothese, gegebenenfalls auch zwischen Prothesenteilen, erlauben. Die Profilierung 9 und die Schraubenlöcher 10 können weitgehend beliebig dem Verwendungszweck angepaßt werden.

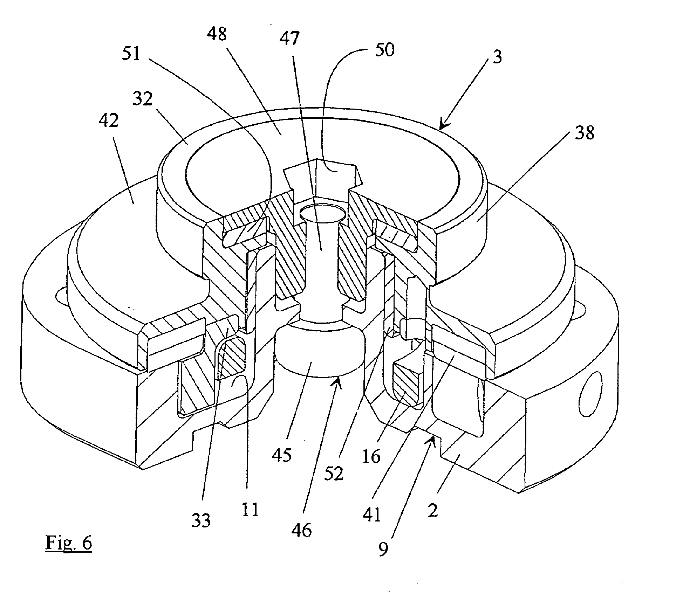

Anhand der Figuren 6 bis 12 wird der innere konstruktive Aufbau des Dämpfers 1 nach der Erfindung weiter erläutert.

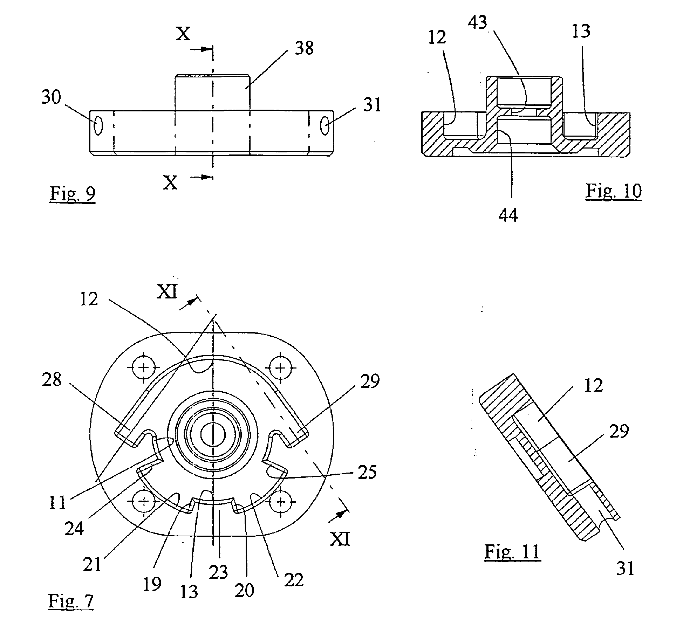

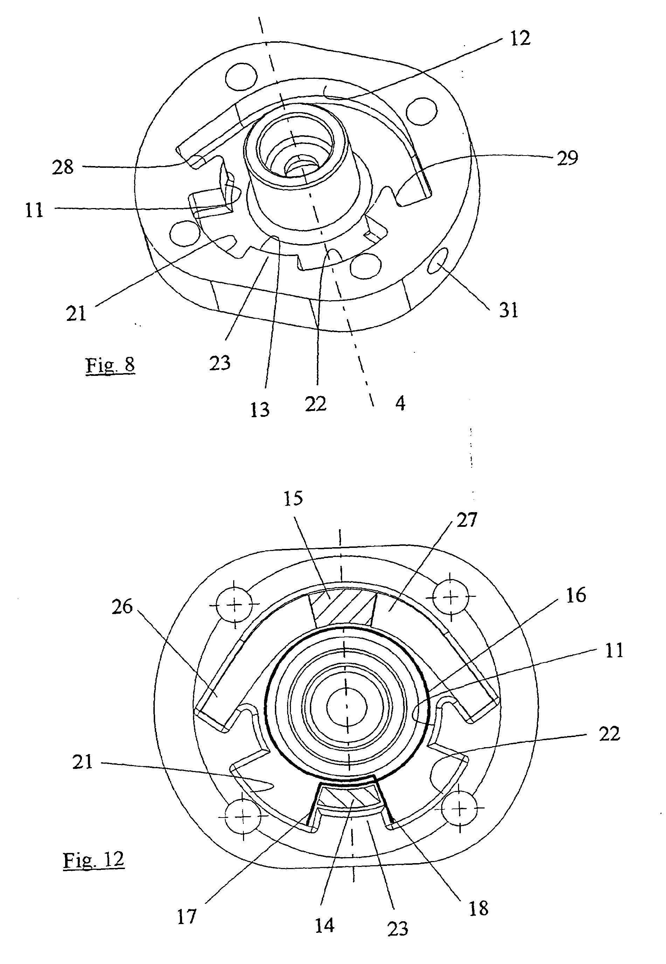

Fig. 7 zeigt in einer Draufsicht auf eine Aufnahme 2 eine bspw. eingefräste oder erodierte, ringförmig umlaufende Nut 11, die zwei Abschnitte 12, 13 aufweist, in der Anschläge 14, 15 des Gehäuseoberteils geführt sind, vgl. auch Fig. 12. In die Nut 11 ist eine Ringfeder 16 die Hochachse 4 umschlingend eingelegt. Die Enden 17, 18 der Ringfeder 16 überkreuzen sich und stützen sich unter Einfassen des Anschlages 14 an Wänden 19, 20 von radial der Nut 11 vorstehenden Taschen 21, 22 ab. Infolge einer Verdrehung des Gehäuseoberteils 3 um die Hochachse 4 gegenüber der Aufnahme 2 bspw. im Uhrzeigersinn wird sich der Anschlag 14 gleichfalls im Uhrzeigersinn in dem Nutabschnitt 13 bewegen. Dabei wird er das in der Darstellung linke Ende 17 der Ringfeder 16 mitnehmen, während sich daß andere rechte Ende 18 weiterhin an der Wand 20 abstützt. D. h. die Drehung erfolgt durch die Kraft der Ringfeder 16 abgebremst.

Nach der Auslenkung und Abbremsung der Drehbewegung wird die in der Ringfeder 16 gespeicherte Kraft das Gehäuseoberteil 3 zurück in seine Neutrallage bringen, in der beide Enden 17, 18 der Feder 16 an den Wänden 19, 20 wieder anliegen, vgl. Fig. 12.

Der Umfangswinkel der Taschen 21, 22 beträgt beim Ausführungsbeispiel etwa 50° jeweils. Der über den Umfang zwischen den Taschen 21, 22 verbleibende Steg 23 mit einem Umfangswinkel von hier etwa 30° bestimmt die Vorspannung der Ringfeder 16. Damit ist eine Vedrehbarkeit des Gehäuseoberteils 3 gegenüber der Aufnahme 2 von maximal etwa 130° gegeben, nämlich aus der Neutrallage in jede Richtung etwa 65°.

Begrenzt wird die Verdrehbarkeit durch Auftreffen der Enden 17, 18 der Ringfeder 16 bzw. des Anschlags 14 selbst auf Wände 24, 25 der Taschen 21, 22, die den Wänden 19, 20 radial gegenüberliegen, an denen sich in der Neutrallage die Enden 17, 18 der Ringfeder 16 abstützen.

Es hat sich als zweckmäßig erwiesen, die hier auftretenden Berührungen der insbesondere metallischen Ringfeder 16, des Anschlags 14 und der Wände 19, 24; 20,25 der Taschen 21; 22 geeignet abzufedern. Dies kann beispielsweise mittels eines elastischen Überzuges der Enden 17, 18 der Ringfeder 16 als Puffer oder auf die Wände 19, 24; 20,25 der Taschen 21; 22 aufgebrachte Puffer, bspw. aus einem Gummi oder einem Kunststoff, erfolgen.

Anhand der oberen Bildhälfte von Fig. 12 wird eine alternative oder wie hier zusätzliche Variante einer Abdämpfung einer Rotationsbewegung weiter erläutert.

In einem Nutabschnitt 12 ist ein zweiter gehäuseoberteilfester Anschlag 15 noch gezeigt. Der Anschlag 15 ist radial zwischen zwei Federelementen 26, 27 spielfrei gehalten, die andernends aufnahmefest angebunden sind. Die Federelemente 26, 27 können Zugfedern bspw. sein, die dann jeweils endseitig fest mit dem Anschlag 15 bzw. der Aufnahme 2 verbunden sein müssen. Es werden deshalb Federelemente 26, 27 bevorzugt, die als Druckfederelemente ausgebildet sind. Die Druckfederelemente 26, 27 können durch metallene Druckfedern dargestellt werden. Bevorzugt werden jedoch stabförmige Druckkörper 26, 27 bspw. aus einem Gummi und insbesondere aus einem Elastomer.

In Verbindung mit einer Abbremsung der Drehbewegung durch die Kraft einer Ringfeder 16 kann die Charakteristik der Dämpfung der Rotation hervorragend eingestellt werden, insbesondere sehr progressiv. Darüberhinaus kann bei geeigneter Materialwahl erreicht werden, daß die Rückführung des Gehäuseoberteils 3 in die Neutrallage durch die in der Ringfeder 16 und in den Federelementen 26, 27 gespeicherte Kraft charakteristisch anders ist. Auch hierdurch wird einem natürlichen Bewegungsablauf Rechnung getragen.

Sind die Federelemente als Druckfederelemente, insbesondere als stabförmige Druckkörper 26, 27 ausgebildet, können sich diese, ohne eine feste Anbindung, auf den radialen Wänden des Anschlages 15 abstützen und werden andernends sich auf geeignet radial ausgebildeten Wänden abstützen können, beispielsweise an den Wänden des Nutabschnittes 12, wenn keine umlaufende ringförmige Nut 11 ausgebildet ist.

Ist, wie dargestellt, eine ringförmig umlaufende Nut 11 für die Aufnahme der Ringfeder 16 vorgesehen, können sich die gezeigten zwei stabförmigen Druckkörper 26, 27 aufnahmefest in an die Nut 11 bzw. an den Nutabschnitt 12 angeschlossene Taschen 28, 29 abstützen, die tangential an den den Anschlag 15 führenden Nutabschnitt 12 angesetzt sind. Die Aufnahme der Ringfeder 16 in der Nut 11 wird dann durch eine Abstützung der Druckkörper 26, 27 nicht beeinträchtigt.

Hierbei ist es weiter von Vorteil, wenn die die zwei Federelemente, die Druckkörper 26, 27 abstützend aufnehmenden Taschen 28, 29 eine einstellbare Tiefe aufweisen. Die Tiefe der Aufnahme ist hier in einfacher, aber effektiver Weise durch Schrauben aufnehmende Durchbrechungen 30, 31 einstellbar, da die Einschraubtiefe und ein Vorstehen des Schaftes der Schraube die Tiefe der Aufnahme bestimmt. Die Durchbrechungen 30, 31 münden jeweils in den Tasche 28, 29 in deren tangentialer Verlängerung. Gegebenenfalls kann zwischen den eingedrehten Enden der Schrauben und den Stirnseiten der Druckkörper 26, 27 noch eine ein Eindringen der Schraubenenden in die Druckkörper 26, 27 verhindernde Distanzplatte eingesetzt sein.

Diese Merkmale eröffnen die Möglichkeit, die Druckkörper 26, 27 in spielfreie radiale Anlage an den entsprechenden Seitenflächen des Anschlages 15 zu bringen und darüber hinaus das Einstellen einer Vorspannung in den Druckkörpern 26, 27 und damit ein individuelles Anpassen der Dämpfung.

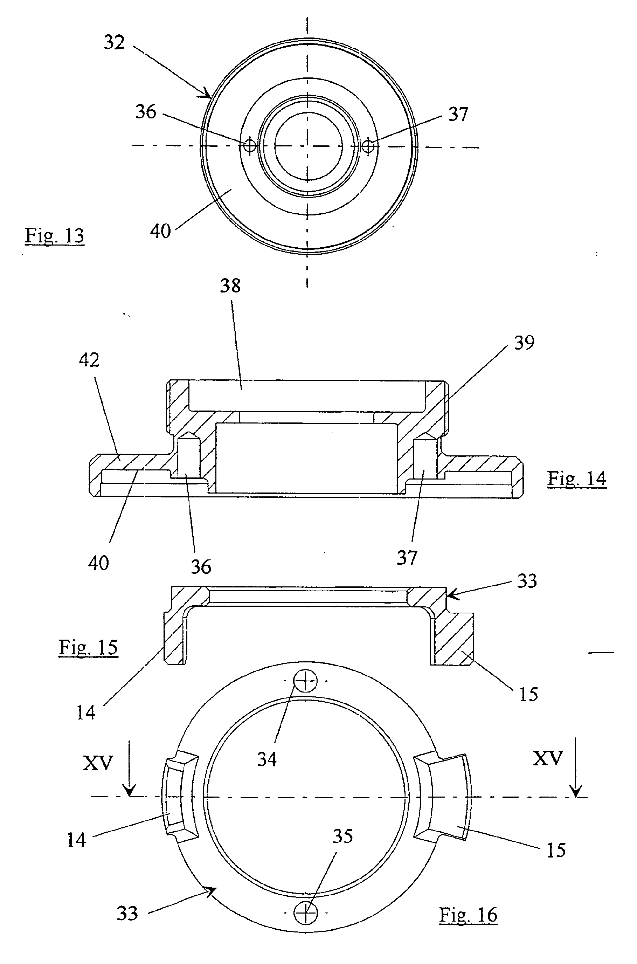

Anhand der Figuren 13 bis 17 wird der Aufbau des Gehäuseoberteils 3 weiter erläutert.

Das Gehäuseoberteil 3 weist einen Gehäusedeckel 32 gem. den Figuren 13 und 14 auf, an den unterseitig ein Ring 33 angeschraubt ist, der die sich diametral gegenüberliegenden Anschläge 14, 15 trägt. Für die Verschraubung weist der Ring 33 Durchbrechungen 34, 35 und der Gehäusedeckel 32 bspw. mit einem Gewinde für die Aufnahme von Schrauben versehene Sackbohrungen 36, 37 auf.

Der Gehäusedeckel 32 weist oberseitig einen axial sich erstreckenden hohlzylindrischen Stutzen 38 auf, der bei dem Ausführungsbeispiel mit einem Außengewinde 39 versehen ist für einen Anschluß an einen Strumpf, ein Prothesenelement oder dergleichen. Alternative Anschlußmöglichkeiten können problemlos vorgesehen werden.

Der Gehäusedeckel ist unterseitig weiter mit einer kreisringförmigen Nut 40 für die Aufnahme eines Axiallagers 41 versehen, vgl. Fig. 6. Bevorzugt wird das Axiallager als Nadellager 41 ausgeführt, das eine geringe Bauhöhe nur verlangt. Es ist die Nut 40 in einer dem übrigen Gehäusedeckel 32 radial vorstehenden Ringschulter 42 eingebracht. Das Axiallager stützt sich hier auf der Oberseite der Aufnahme 2 weiter dann ab. Der exakten Führung bei einer Rotation dient weiter eine Buchse 52.

Anhand der Figuren 6 bis 8 wird der axialen Zusammenhalt von Aufnahme 2 und Gehäuseoberteil 3 nachstehend erläutert. Die Aufnahme 2 weist eine zentrale Durchbrechung 43 auf. Unterseitig der Aufnahme 2 ist die Durchbrechung 44 zu einer Aufnahme 44 für die Aufnahme eines Schraubenkopfes 45 einer Zylinderschraube 46 aufgeweitet, vgl. Fig. 6, deren Schaft 47 die Durchbrechung 43 durchsetzt.

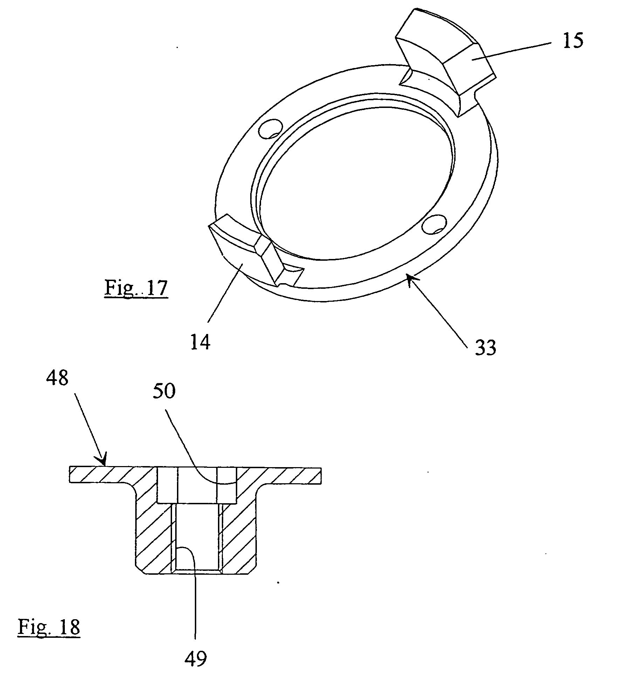

In den hohlzylindrischen Stutzen 38 des Gehäusedeckels 32 ist von oben ein Deckel 48 gem. Fig. 18 eingesetzt, aufliegend auf einer Scheibe 51, der gleichfalls eine zentrale Durchbrechung 49 aufweist für die Aufnahme des Schaftes 47 der Zylinderschraube 46. In einem oberen Abschnitt weitet sich die Durchbrechung 49 zu einer Aufnahme 50 einer die Zylinderschraube 46 konternde Mutter auf.

### Bezugszeichenliste:

- 1.: Dämpfer
- 2.: Aufnahme
- 3.: Gehäuseoberteil
- 4.: Hochachse
- 5.: Rechteck
- 6.: Abschnitt
- 7.: Abschnitt
- 8.: Abfasung,
Abrundung
- 9.: Profilierung
- 10.: Schraubenloch
- 11.: Nut
- 12.: Abschnitt von 11
- 13.: Abschnitt von 11
- 14.: Anschlag
- 15.: Anschlag
- 16.: Ringfeder
- 17.: Ende von 16
- 18.: Ende von 16
- 19.: Wand von 21
- 20.: Wand von 22
- 21.: Tasche
- 22.: Tasche
- 23.: Steg
- 24.: Wand von 21
- 25.: Wand von 22
- 26.: Federelement
- 27.: Federelement
- 28.: Tasche
- 29.: Tasche
- 30.: Durchbrechung
- 31.: Durchbrechung
- 32.: Gehäusedeckel
- 33.: Ring
- 34.: Durchbrechung
- 35.: Durchbrechung
- 36.: Sackbohrung
- 37.: Sackbohrung
- 38.: Stutzen
- 39.: Außengewinde
- 40.: Nut
- 41.: Nadellager
- 42.: Ringschulter
- 43.: Durchbrechung
- 44.: Aufnahme
- 45.: Schraubenkopf
- 46.: Zylinderschraube
- 47.: Schaft
- 48.: Deckel
- 49.: Durchbrechung
- 50.: Aufnahme
- 51.: Scheibe
- 52.: Buchse

## Patentansprüche

1. Dämpfer für Rotationsbewegungen bei Prothesen, aufweisend eine untere Aufnahme (2) und ein gegen die Kraft eines Federelements gegenüber der Aufnahme (2) um eine Hochachse (4) verdrehbares Gehäuseoberteil (3) **gekennzeichnet durch** wenigstens einen gehäuseoberteilfesten, zwei radiale Anschlagflächen ausbildenden Anschlag (14; 15), an die wenigstens ein an der Aufnahme (2) festgelegtes Federelement (16; 26, 27) angeschlossen ist, dessen Kraft die Drehbewegung dämpft und das Gehäuseoberteil (3) nach einem Verdrehen gegenüber der Aufnahme (2) in eine Neutrallage zurückführt.

2. Dämßpfer nach Anspruch 1, **dadurch gekennzeichnet, daß** der Anschlag (14) in einer zumindest abschnittsweise ringartigen Nut (12, 13; 11) in der Aufnahme (2) geführt ist.

3. Dämpfer nach einem oder mehreren der vorangehenden Ansprüche, **dadurch gekennzeichnet, daß** in eine ringartig umlaufende Nut (11) eine Ringfeder (16) eingelegt ist, deren Enden (17, 18) unter Einfassen des Anschlages (14) sich in einer Neutrallage überkreuzend an Wänden (19, 20) von radial der Nut (11) vorstehenden Taschen (21, 22) abstützen.

4. Dämpfer nach einem oder mehreren der vorangehenden Ansprüche, **dadurch gekennzeichnet, daß** das Einfassen des Anschlages (14) durch elastische Puffer gedämpft erfolgt.

5. Dämpfer nach einem oder mehreren der vorangehenden Ansprüche, **dadurch gekennzeichnet, daß** das Abstützen der Enden (17, 18) der Ringfeder (16) durch elastische Puffer gedämpft erfolgt.

6. Dämpfer nach einem oder mehreren der vorangehenden Ansprüche, **dadurch gekennzeichnet, daß** ein Anschlag (15) radial zwischen zwei Federelementen (26, 27) spielfrei gehalten ist, die andernends aufnahmefest angebunden sind.

7. Dämpfer nach einem oder mehreren der vorangehenden Ansprüche, **dadurch gekennzeichnet, daß** die zwei Federelemente (26, 27) Druckfederelemente sind.

8. Dämpfer nach einem oder mehreren der vorangehenden Ansprüche, **dadurch gekennzeichnet, daß** die zwei Federelemente (26, 27) aus einem Elastomere bestehende Druckkörper sind.

9. Dämpfer nach einem oder mehreren der vorangehenden Ansprüche, **dadurch gekennzeichnet, daß** sich die zwei Federelemente (26, 27) in an die Nut (11) angeschlossene Taschen (28, 29) abstützen.

10. Dämpfer nach einem oder mehreren der vorangehenden Ansprüche, **dadurch gekennzeichnet, daß** die die zwei Federelemente (26, 27) abstützenden Taschen (28, 29) tangential an den den Anschlag (15) führenden Nutabschnitt (12) angesetzt sind.

11. Dämpfer nach einem oder mehreren der vorangehenden Ansprüche, **dadurch gekennzeichnet, daß** die die zwei Federelemente (26, 27) abstützende Taschen (28, 29) eine einstellbare Tiefe aufweisen.

12. Dämpfer nach einem oder mehreren der vorangehenden Ansprüche, **dadurch gekennzeichnet, daß** die Aufnahme (2) jeweils eine in einer Tasche (28, 29) in tangentialer Verlängerung mündende Durchbrechung (30, 31) für die Aufnahme einer Schraube aufweist, deren Eindrehtiefe in die Durchbrechung (30, 31) die Tiefe der Tasche (28, 29) bestimmt.

13. Dämpfer nach einem oder mehreren der vorangehenden Ansprüche, **dadurch gekennzeichnet, daß** am Gehäuseoberteil (3) zwei sich diametral gegenüberliegende Anschläge (14, 15) vorgesehen sind, von denen einer (15) radial zwischen zwei Federelementen (26, 27) spielfrei gehalten ist und der andere (14) zwischen den sich überkreuzenden Enden (17, 18) einer Ringfeder (16).

14. Dämpfer nach einem oder mehreren der vorangehenden Ansprüche, **dadurch gekennzeichnet, daß** das Gehäuseoberteil (3) mittels eines Axiallagers (41) an der Aufnahme (2) drehbar gelagert ist.

15. Dämpfer nach einem oder mehreren der vorangehenden Ansprüche, **dadurch gekennzeichnet, daß** das Axiallager (41) ein Nadellager ist.

## Claims

1. Damper for rotational movements in prostheses, having a lower holder (2) and an upper housing part (3) that can be rotated with respect to the holder (2) about a vertical axis (4) against the force of a spring element, **characterized by** at least one stop (14; 15), fixed to the upper housing part, forming two radial stopping surfaces, to which at least one spring element (16; 26; 27) rigidly fixed on the holder (2) is connected, the force of which damps the rotational motion and guides the upper housing part (3) back into a neutral position after a rotation with respect to the holder (2).

2. Damper according to Claim 1, **characterised in that** the stop (14) is guided in the holder (2) in a groove (12, 13; 11) that is annular at least in some sections.

3. Damper according to one or a multiple of the preceding claims, **characterized in that**, an annular spring (16) is engaged into an annular circumferential groove (11), the ends (17, 18) of which, by enclosing the stop (14), are supported in a neutral position intersecting at walls (19, 20) of slots (21, 22) protruding radially from the groove (11).

4. Damper according to one or a multiple of the preceding claims, **characterized in that**, the enclosure of the stop (14) is damped by means of elastic buffers.

5. Damper according to one or a multiple of the preceding claims, **characterized in that**, the supporting of the ends (17, 18) of the annular spring (16) is damped by means of elastic buffers.

6. Damper according to one or a multiple of the preceding claims, **characterized in that**, a stop (15) is held radially free of play between two spring elements (26, 27), which are rigidly fixed to the holder at the other ends of the elements.

7. Damper according to one or a multiple of the preceding claims, **characterized in that**, the two spring elements (26, 27) are compression spring elements.

8. Damper according to one or a multiple of the preceding claims, **characterized in that**, the two spring elements (26, 27) are compression bodies consisting of an elastomer.

9. Damper according to one or a multiple of the preceding claims, **characterized in that**, the two spring elements (26, 27) are supported in slots (28, 29) connected to the groove (11).

10. Damper according to one or a multiple of the preceding claims, **characterized in that**, that the two slots (28, 29) supporting the two spring elements (26, 27) are attached tangentially to the groove section (12) guiding the stop (15).

11. Damper according to one or a multiple of the preceding claims, **characterized in that**, the two slots (28, 29) supporting the two spring elements (26, 27) have an adjustable depth.

12. Damper according to one or a multiple of the preceding claims, **characterized in that**, the holder (2) has through holes (30, 31) respectively opening into a slot (28, 29) in a tangential elongation for holding a screw, the penetration depth of which into the through hole (30, 31) determines the depth of the slot (28, 29) .

13. Damper according to one or several of the foregoing claims, **characterized in that**, on the upper housing part (3), two diametrically opposite stops (14, 15) are provided, one (15) of which is held radially free of play between two spring elements (26, 27), and the other (14) between the intersecting ends (17, 18) of an annular spring (16).

14. Damper according to one or a multiple of the preceding claims, **characterized in that**, the upper housing part (3) is rotationally mounted on the holder (2) by means of an axial bearing (41).

15. Damper according to one or a multiple of the preceding claims, **characterized in that**, that the axial bearing (41) is a needle bearing.

## Revendications

1. Amortisseur de mouvements rotatoires sur des prothèses, comportant un logement inférieur (2) et une partie supérieure de boîtier (3) rotative autour d'un axe normal (4), par rapport au logement (2) contre la force d'un élément à ressort, **caractérisé par** au moins une butée (14; 15) stationnaire sur la partie supérieure du boîtier, formant deux surfaces de butée radiales, sur lesquelles est raccordé au moins un élément à ressort (16; 26, 27) fixé sur le logement (2), dont la force amortit la rotation et ramène la partie supérieure de boîtier (3) dans une position neutre, après une torsion par rapport au logement (2).

2. Amortisseur selon la revendication 1, **caractérisé en ce que** la butée (14) est guidée dans le logement (2) dans une rainure (12, 13 ; 11), au moins circulaire par sections.

3. Amortisseur selon l'une quelconque ou plusieurs des revendications précédentes, **caractérisé en ce que**, dans une rainure (11) s'étendant sous forme annulaire, est inséré un anneau-ressort (16) dont les extrémités (17, 18) s'appuient en entourant la butée (14), en se croisant dans une position neutre sur des parois (19, 20) de poches (21, 22) saillant latéralement à partir de la rainure (11).

4. Amortisseur selon l'une quelconque ou plusieurs des revendications précédentes, **caractérisé en ce que** l'entourage de la butée (14) est assuré de façon amortie par des tampons élastiques.

5. Amortisseur selon l'une quelconque ou plusieurs des revendications précédentes, **caractérisé en ce que** l'appui des extrémités (17, 18) de l'anneau-ressort (16) est assuré de façon amortie par des tampons élastiques.

6. Amortisseur selon l'une quelconque ou plusieurs des revendications précédentes, **caractérisé en ce qu'**une butée (15) est maintenue sans jeu, en direction radiale entre deux éléments à ressort (26, 27), qui par leur autre extrémité sont reliés fixement dans le logement.

7. Amortisseur selon l'une quelconque ou plusieurs des revendications précédentes, **caractérisé en ce que** les deux éléments à ressort (26, 27) sont des éléments à ressort de pression.

8. Amortisseur selon l'une quelconque ou plusieurs des revendications précédentes, **caractérisé en ce que** les deux éléments à ressort (26, 27) sont en un élément de pression en un élastomère.

9. Amortisseur selon l'une quelconque ou plusieurs des revendications précédentes, **caractérisé en ce que** les deux éléments à ressort (26, 27) s'appuient dans des poches (28, 29) raccordées sur la rainure (11).

10. Amortisseur selon l'une quelconque ou plusieurs des revendications précédentes, **caractérisé en ce que** les poches (28, 29) sur lesquelles s'appuient les deux éléments à ressort (26, 27) sont raboutées de façon tangentielle sur la section de rainure (15) guidant la butée (12).

11. Amortisseur selon l'une quelconque ou plusieurs des revendications précédentes, **caractérisé en ce que** les poches (28, 29) sur lesquelles s'appuient les deux éléments à ressort (26, 27) présentent une profondeur réglable.

12. Amortisseur selon l'une quelconque ou plusieurs des revendications précédentes, **caractérisé en ce que** le logement (2) présente chaque fois un évidement (30, 31) débouchant en prolongement tangentiel dans une poche (28, 29) pour le logement d'une vis, dont la profondeur de vissage dans l'évidement (30, 31) définit la profondeur de la poche (28, 29).

13. Amortisseur selon l'une quelconque ou plusieurs des revendications précédentes, **caractérisé en ce que** sur la partie supérieure du boîtier (3) sont prévues deux butées (14, 15) diamétralement opposées, dont l'une (15) est maintenue sans jeu en direction radiale entre deux éléments à ressort (26, 27) et l'autre (14) entre les extrémités qui se croisent (17, 18) d'un anneau-ressort (16).

14. Amortisseur selon l'une quelconque ou plusieurs des revendications précédentes, **caractérisé en ce que** la partie supérieure du boîtier (3) est logée de façon rotative sur le logement (2) au moyen d'un palier axial (41).

15. Amortisseur selon l'une quelconque ou plusieurs des revendications précédentes, **caractérisé en ce que** le palier axial (41) est un roulement à aiguilles.
